Europäisches Patentamt

European Patent Office

Office européen des brevets

(19)

(11) Publication number: **0 078 061**
**B1**

(12) **EUROPEAN PATENT SPECIFICATION**

(45) Date of publication of patent specification: **11.02.87**

(51) Int. Cl.⁴: **C 12 N 5/02**

(21) Application number: **82109939.7**

(22) Date of filing: **27.10.82**

(54) **Method for culturing non-adhering mammalian cells.**

(30) Priority: **28.10.81 JP 172265/81**

(43) Date of publication of application:
**04.05.83 Bulletin 83/18**

(45) Publication of the grant of the patent:
**11.02.87 Bulletin 87/07**

(84) Designated Contracting States:
**CH DE FR GB LI**

(56) References cited:
**FR-A-2 236 004**
**CHEMICAL ABSTRACTS, vol. 70, no. 11, 17th
March 1969, page 145, no. 45492y, Columbus,
Ohio, USA.**
**CHEMICAL ABSTRACTS, vol. 88, no. 25, 19th
June 1978, page 458, no. 187063e, Columbus,
Ohio, USA**
**BIOLOGICAL ABSTRACTS, vol. 68, 1979, no.
46467**

**The file contains technical information
submitted after the application was filed and
not included in this specification**

(73) Proprietor: **AJINOMOTO CO., INC.
5-8, Kyobashi 1-chome, Chuo-ku
Tokyo 104 (JP)**

(72) Inventor: **Shibai, Hiroshiro
No. 14-15, Wakamatsu-cho
Chigasaki-shi Kanagawa-ken (JP)**
Inventor: **Nishimura, Naoki
No. 2-20-8, Kannon Kawasaki-ku
Kawasaki-shi Kanagawa-ken (JP)**
Inventor: **Nakamatsu, Tsuyoshi
No. 1516-3, Seya-cho Seya-ku
Yokohama-shi Kanagawa-ken (JP)**
Inventor: **Takano, Satoshi
No. 1740-66, Kamigo-cho Totsuka-ku
Yokohama-shi Kanagawa-ken (JP)**

(74) Representative: **Schübel-Hopf, Ursula et al
Strehl, Schübel-Hopf, Schulz Patentanwälte
Widenmayerstrasse 17
D-8000 München 22 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**0 078 061**

(56) References cited:

CHEMICAL ABSTRACTS, vol. 87, no. 21, 21st
November 1977, page 400, no. 165762j,
Columbus, Ohio, USA

NATURE, vol. 267, 2nd June 1977, pages
423-425. L. PACKER et al.: "Low oxygen
concentration extends the lifespan of cultured
human diploid cells"

CHEMICAL ABSTRACTS, vol. 75, no. 9, 30st
August 1971, page 178, no. 60514d, Columbus,
Ohio, USA

NATURE, vol. 182, 29th November 1958, pages
1508-1509. P. D. COOPER et al.: "Critical effect
of Oxygen tension on rate of growth of animal
cells in continuous suspended culture"

# 0 078 061

**Description**

The present invention relates to a method for culturing non-adhering mammalian cells, and particularly relates to a method for culturing non-adhering mammalian cells in an aqueous culture medium in suspension.

It is well known that most of non-adhering mammalian cells can propagate in aqueous media in suspension. Known mammalian cells capable of propagating in suspension are, for example, lymphoblastoid cell such as T and B lymphoblastoma, myeloma cell such as IgD myelomana and other leukemia (e.g. myelomonocytic cell), and mammary carcinoma cell ("Gane" Youichi Yamamura et al (ed.), Kyoritsu Shuppan (1978).

Recently, it has been reported that such suspension cultivation of mammalian cells are applicable to the production of lymphokain and interferon, and the technique has became very important.

In known suspension cultivation of mammalian cells, oxygen necessary for the propagation of cells is supplied by a rotating or turning culture vessel, or agitating or stirring means in the vessel. Usual cultivation vessels are closed vessels, vessels with a gas-permeable plug such as cotton plug and vessels with aeration and agitation means.

Only a few reports are found as to the relationship between oxygen supply and cell growth in suspension cultivation such as P. D. Cooper et al., Nature, *182*, 1508 (1958), in which it is reported that the growth rate of kidney cells of rabbit is high in case of an amount of oxygen in the gas phase from 0.17 to 0.19 atm when the cells are cultured in suspension under various levels of oxygen in the gas phase ranging from 0.14 atm to 0.22 atm.

As explained above, the knowledge about suspension cultivation of mammalian cells is insufficient, and further development in suspension cultivation of non-adhering mammalian cells- is required for industrial cultivation of mammalian cells.

The inventors have perceived the importance of dissolved oxygen in aqueous culture medium for the growth of mammalian cells, and obtained excellent growth of mammalian cells when the cells are cultured under a partial pressure of dissolved oxygen lower than that to be expected according to known facts. That is, the inventors cultured a lymphoblastoid cell derived from human umbilical cord blood lymphocyte in a closed culture vessel to study the relationship between cell growth and dissolved oxygen, and found that the partial pressure of dissolved oxygen rapidly fell below 0.01 atm after the partial pressure of dissolved oxygen reached at 0.1 atm at a late stage of exponential growth phase, and cell growth ceased at the time. However, when the lymphoblastoid cells were cultured with a sufficient aeration, the partial pressure of dissolved oxygen became 0.1 to 0.2 atm and good growth was obtained but still lower than what desired.

Based on these findings, the inventors have studied further and found that cell growth was excellent, when non-adhering mammalian cells were cultured at a partial pressure of dissolved oxygen below 0.05 atm until the middle stage of exponential growth phase, and the minimum value of dissolved oxygen necessary for the cell growth was 0.01 atm.

It has now been provided a method for culturing non-adhering mammalian cell which comprises culturing non-adhering mammalian cells in an aqueous culture medium in suspension at a partial pressure of dissolved oxygen in the range from 0.01 to 0.05 bars (atm) until the middle stage of exponential growth phase, and at a partial pressure of dissolved oxygen of more than 0.01 bars (atm) after the middle stage of exponential growth phase.

In the following preferred embodiments of the invention are described.

Non-adhering mammalian cells cultured by the method of the present invention are those which can grow in an aqueous medium without adhering at the wall of the culture vessel, and include:

a) Lymphoblastoid cells: RPMI 1788 (Moore, G. E., et. al., J. Nat. Cancer Inst., *43*, 1119 (1969)), MOLT-4B (Minowada, J., et. al., J. Nat. Cancer Inst., *49*, 891 (1972)), BALL (Miyoshi, I., et. al., Nature, *267*, 843 (1977)), NALL-1 (Hiraki, S., et. al., Cancer, *40*, 2131 (1977)), P13/Koyama (Minato, K., et. al., Recent Advances in RES' Research, *17*, 116 (1977)).

b) Lymphoblastoma cells: CCRF-CEM (Foley, G. E., et. al., Cancer, *18*, 522 (1965)), CCRF-SB (Foley, G. E., et. al., M. D. Anderson Hospt. Tumor Inst. Mongr., *21*, (1967)).

c) Myeloma cells: RPMI 8226 (Moore, G. E., et. al., New York State J. Med., *66*, 2757 (1966)).

d) Mammary carcinoma: HPL-MmC (Morikawa, S., Proceedings of the Japanese Cancer Association, the 34th Annual Meeting, p. 72 (1975)).

The partial pressure of dissolved oxygen can be determined by any conventional manner such as using dissolved oxygen electrode.

In order to control the partial pressure of dissolved oxygen, any known method can be used, and most simple methods are, for example, to control agitation speed, inner pressure of the cultivation vessel and partial pressure of oxygen in gas to be introduced into the cultivation vessel.

The middle stage of exponential growth phase of the present invention is the time when the cell number of non-adhering mammalian cell becomes A in the following equation:

$$1/2 \log XY - 1/6 \log Y/X < \log A < 1/2 \log XY + 1/6 \log Y/X$$

In the above equation, X is the initial cell number and Y is the maximum cell number. The maximum

3

cell number Y can be obtained by culturing non-adhering mammalian cells at 0.05 atm of partial pressure of dissolved oxygen using previously determined aqueous culture medium and culture conditions.

The cell number can be counted using Thoma hematometer or trypan blue dye exclusion method.

The partial pressure of dissolved oxygen in the aqueous culture medium is controlled at a level ranging from 0.01 to 0.05 atm and more preferably from 0.02 to 0.05 atm until middle stage of exponential growth phase. After the middle stage of exponential growth phase, the partial pressure of dissolved oxygen is maintained at a level higher than 0.01 atm and preferably higher than 0.02 atm. Desirably, the partial pressure of dissolved oxygen is maintained at a level below 0.2 atm after the middle stage of exponential growth phase.

The aqueous culture media employed in the present invention are, for example, E-MEM (Eegle, H., Science, *130*, 432 (1959)), D-MEM (Dulbecco, R. and Freeman, G., Virology, *8*, 396 (1959), RPMI-1640 (Moore, G. E. et. al., J. A. M. A., *199*, 519 (1967) and RITC 55-9 (Yamane, I. et. al., Exp. Cell Res., *134*, 470 (1981)).

Cultivation of the mammalian cells is conducted in a conventional manner such as turning a cylinder inclined with the angle of 5° in which test tubes are set (Earle, W. R., et. al., J. Nat. Cancer Inst., *14*, 853 (1954)), flasks rotating along a horizontal circumference (Earle, W. R., Fed. Proc., *17*, 967 (1957)) and agitating with a magnetic stirrer put in cultivation vessel (spinner cultivation) (McLimans, W. F., et. al., J. Immunol., *79*, 428 (1957).

The cultivation temperature is usually adjusted to 36° to 39°C.

The pH of the aqueous culture medium is preferably maintained at pH 6.8 to 7.6 and more preferably 7.2 to 7.4, by ventilating gas-phase in cultivation vessel with air containing carbon dioxide, using media containing sodium bicarbonate, or adding gaseous carbon-dioxide into the gas-phase in a closed cultivation vessel.

Example 1

A lymphoblastoid cell line derived from human umbilical cord blood lymphocyte was suspended in 700 ml batches of RITC 55-9 medium placed in 1,000 ml volume-glass-spinner bottles (Bellco Glass, Inc.) which was equipped with a dissolved oxygen sensor, to contain 4 to $5 \times 10^5$ cell per milliliter. Cultivation of the cell was carried out with 150 rpm agitation while ventilating the gas-phase in the bottle with an air containing 5% of carbon dioxide to maintain the pH of the medium to 7.0 to 7.4.

Partial pressure of dissolved oxygen (D.O) in the medium was controlled, from initial stage of cultivation to middle stage of exponential growth phase, in the range from 0 to 0.01 atm, from 0.01 to 0.05 atm, from 0.05 to 0.10 atm or 0.1 to 0.2 atm by changing flow rate and ratio of oxygen or nitrogen concentration in ventilating gas.

After the middle stage of exponential growth phase, the partial pressure of dissolved oxygen was controlled above 0.01 atm.

In the course of the cultivation, cells were counted.

α-Interferon was produced proportionally to the growth of cells.

The process by which the lymphoblastoid cell used in the present Example had been derived from human umbilical cord blood lymphocyte is disclosed in "Proceeding of the International Conference on Clinical Potentials of Interferons in Viral Diseases and Malignant Tumors", held December 2—4, 1980, and precisely in Yamane, I., et. al., "Spontaneous and Potential Interferon Producing Cell Lines Transformed from Human Cord Blood Lymphocytes and Grown in Serum-free Medium", in "The Clinical Potential of Interferons", (Univ. of Tokyo Press, Tokyo, Japan), pp. 87—100, 1982. The lymphoblastoid cell line was delivered by Dr. Isao Yamane, one of the authors of the above literatures.

Previously determined maximum cell number, which had been determined by culturing the lymphoblastoid by the manner shown above except that partial pressure of dissolved oxygen was adjusted to 0.05 atm, was $2.4 \times 10^6$. Therefore, 1/2 log XY − 1/6 log Y/X was 5.85, and 1/2 log XY + 1/6 log Y/X was 6.10. The cell number at the middle stage of exponential growth phase was $1.1 \times 10^6$ and then log A is 6.04.

Example 2

In the experiment in Example 1, partial pressure of dissolved oxygen (D.O.) was maintained in the range from 0.01 to 0.05 atm until middle stage of exponential growth phase (24 hours from initiation), and after the middle stage of exponential growth phase, partial pressure of dissolved oxygen was maintained in the range shown in Table 1. In the course of the cultivation, cells were counted and the results are shown in Table 1.

**0 078 061**

TABLE 1

| D.O. after the middle stage of exponential growth phase (atm) | Cell number (per ml) | | | |
|---|---|---|---|---|
| | Cultivation times (hr) | | | |
| | 0 | 24 | 48 | 72 |
| 0—0.01 | $4.4\times10^5$ | $7.4\times10^5$ | $5.0\times10^5$ | $1.0\times10^6$ |
| 0.01—0.05 | $4.2\times10^5$ | $7.5\times10^5$ | $1.4\times10^6$ | $2.0\times10^6$ |
| 0.05—0.10 | $4.4\times10^5$ | $7.6\times10^5$ | $1.4\times10^6$ | $1.9\times10^6$ |
| 0.10—0.20 | $4.0\times10^5$ | $7.3\times10^5$ | $1.4\times10^5$ | $1.9\times10^6$ |

α-Interferon was produced proportionally to the growth of cell.

Cell number of the middle stage of exponential growth phase was 7.3—$7.6\times10^5$ (A), and log A is $5.86\sim5.88$.

Example 3

CCRF-CEM, a human T-lymphoblastoma (Foley, G. F. et. al., Cancer, *18*, 522 (1965), which was sold by DAI-ICHI SEIYAKU Co., Japan, was suspended in 1,000 ml batches of RPMI-1640 containing 10% fetal bovine serum and placed in 1,000 ml volume-glass-spinner bottles (Belleco Glass Inc.) which were equipped with dissolved oxygen electrode sensor, to contain 1—$1.1\times10^5$ cells per milliliter, and were cultured by the manner shown in Example 1 except that agitation speed was 100 rpm.

TABLE 2

| D.O. before the middle stage of exponential growth (atm) | D.O. after the middle stage of exponential growth (atm) | Cell number (per ml) | | | |
|---|---|---|---|---|---|
| | | Cultivation time (hr) | | | |
| | | 0 | 24 | 48 | 72 |
| 0.01—0.05 | 0—0.01 | $1.1\times10^5$ | $2.5\times10^5$ | $3.0\times10^5$ | $8.0\times10^4$ |
| " | 0.01—0.05 | $1.0\times10^5$ | $2.3\times10^5$ | $5.6\times10^5$ | $1.4\times10^6$ |
| " | 0.05—0.10 | $1.1\times10^5$ | $2.4\times10^5$ | $5.7\times10^5$ | $1.4\times10^6$ |
| 0.05—0.10 | 0—0.01 | $1.2\times10^5$ | $2.0\times10^5$ | $2.5\times10^5$ | $4.0\times10^4$ |
| " | 0.01—0.05 | $1.0\times10^5$ | $2.1\times10^5$ | $4.8\times10^5$ | $1.1\times10^6$ |
| " | 0.05—0.10 | $1.1\times10^5$ | $2.1\times10^5$ | $4.9\times10^5$ | $1.0\times10^6$ |
| 0.15—0.20 | 0.01—0.05 | $1.0\times10^5$ | $1.7\times10^5$ | $4.2\times10^5$ | $8.7\times10^5$ |
| " | 0.15—0.20 | $1.1\times10^5$ | $1.7\times10^5$ | $4.0\times10^5$ | $8.0\times10^5$ |

In this experiment, previously determined maximum cell number was $1.4\times10^6$, and 1/2 log XY−1/6 log Y/X is 5.39 and 1/2 log XY−1/6 log Y/X is 5.75.

Partial pressure of dissolved oxygen (D.O.) was maintained in the range from 0.01 to 0.05 atm, from 0.05 to 0.10 atm or from 0.15 to 0.20 atm, until the middle stage of exponential growth phase, 35 hours from the initiation (cell number was $2.6\times10^5$ to $3.6\times10^5$ and log A is from 5.41 to 5.56), and after the middle stage of exponential growth phase, D.O. was maintained in the ranges shown in Table 2. In the course of the culture, cells were counted and the results are shown in Table 2.

Example 4

CCRF-CEM was suspended to contain 1 to $1.5\times10^5$ cell per milliliter, in 1,000 ml batches of RPMI-1640 medium containing 10% fetal bovine serum which were placed in spinner flasks (Bellco Glass Inc.) equipped with dissolved oxygen sensor. Cultivation was conducted by the manner shown in Example 3.

5

**0 078 061**

The partial pressure of dissolved oxygen was adjusted to a level in the range from 0.01 to 0.05 atm until the cell number became that shown in Table 3, and thereafter partial pressure of dissolved oxygen was rised to a level in the range from 0.15 to 0.20 atm. Cell numbers were counted after the cultivation and are shown in Table 3.

TABLE 3

| Time D.O. rised | | | Cell number (per ml) | | | |
|---|---|---|---|---|---|---|
| | | | Cultivation time (hr) | | | |
| Cell number (/ml) | Cultivation time (hr) | log A | 0 | 24 | 48 | 72 |
| $1.0 \times 10^5$ | 0 | 5.00 | $1.0 \times 10^5$ | $1.8 \times 10^5$ | $4.2 \times 10^5$ | $8.1 \times 10^5$ |
| $1.6 \times 10^5$ | 14 | 5.20 | $1.1 \times 10^5$ | $2.2 \times 10^5$ | $4.9 \times 10^5$ | $1.0 \times 10^6$ |
| $2.5 \times 10^5$ | 24 | 5.40 | $1.2 \times 10^5$ | $2.5 \times 10^5$ | $5.9 \times 10^5$ | $1.4 \times 10^6$ |
| $4.8 \times 10^5$ | 42 | 5.68 | $1.2 \times 10^5$ | $2.3 \times 10^6$ | $5.7 \times 10^5$ | $1.4 \times 10^6$ |
| $8.0 \times 10^5$ | 60 | 5.90 | $1.0 \times 10^5$ | $2.4 \times 10^5$ | $5.8 \times 10^5$ | $1.4 \times 10^6$ |

## Claims

1. A method for culturing non-adhering mammalian cells, which comprises culturing non-adhering mammalian cells in an aqueous culture medium in suspension at a partial pressure of dissolved oxygen in the range from 0.01 bars (atm) to 0.05 bars (atm) until the middle stage of the exponential growth phase, and at a partial pressure of dissolved oxygen of more than 0.01 bars (atm) after the middle stage of exponential growth phase.

2. The method of Claim 1, wherein said non-adhering mammalian cells are cultured at a partial pressure of dissolved oxygen in the range from 0.02 to 0.05 bars (atm) until the middle stage of the exponential growth phase.

3. The method of Claim 1 or Claim 2, wherein said non-adhering mammalian cells are cultured at a partial pressure of dissolved oxygen lower than 0.2 bars (atm) after the middle stage of exponential growth phase.

4. The method of Claim 1, 2 or 3, wherein said non-adhering mammalian cells are cultured at a partial pressure of dissolved oxygen higher than 0.02 bars (atm) after the middle stage of the exponential growth phase.

## Patentansprüche

1. Verfahren zur Züchtung von nichthaftenden Säugetierzellen, darin bestehend, daß nichthaftende Säugetierzellen in Suspension in einem wässrigen Kulturmedium bei einem Partialdruck an gelöstem Sauerstoff im Bereich von 0,01 bar (atm) bis 0,05 bar (atm) bis zum mittleren Stadium der exponentiellen Wachstumsphase, und bei einem Partialdruck an gelöstem Sauerstoff von mehr als 0,01 bar (atm) nach dem mittleren Stadium der exponentiellen Wachstumsphase gezüchtet werden.

2. Verfahren gemäß Anspruch 1, bei dem die nichthaftenden Säugetierzellen bis zum mittleren Stadium der exponentiellen Wachstumsphase bei einem Partialdruck an gelöstem Sauerstoff, der in dem Bereich von 0,02 bis 0,05 bar (atm) liegt, gezüchtet werden.

3. Verfahren gemäß einem der Ansprüche 1 oder 2, bei dem die nichthaftenden Säugetierzellen nach dem mittleren Stadium der exponentiellen Wachstumsphase bei einem Partialdruck an gelöstem Sauerstoff, der niedriger ist als 0,2 bar (atm), gezüchtet werden.

4. Verfahren gemäß einem der Ansprüche 1, 2 oder 3, bei dem die nichthaftenden Säugetierzellen nach dem mittleren Stadium der exponentiellen Wachstumsphase bei einem Partialdruck an gelöstem Sauerstoff, der höher ist als 0,02 bar (atm), gezüchtet werden.

## Revendications

1. Un procédé de culture de cellules non adhérentes de mammifères qui comprend la culture des cellules non adhérentes de mammifères en suspension dans un milieu de culture aqueux à une pression partielle de l'oxygène dissous dans la gamme de 0,01 bar (atmosphère) à 0,05 bar (atmosphère) jusqu'au stade moyen de la phase de croissance exponentielle et à une pression partielle de l'oxygène dissous supérieure à 0,01 bar (atmosphère) après la phase moyenne de la croissance exponentielle.

6

2. Le procédé de la revendication 1 dans lequel les cellules non adhérentes de mammifères sont cultivées à une pression partielle de l'oxygène dissous dans la gamme de 0,02 à 0,05 bar (atmosphère) jusqu'au stade moyen de la phase de croissance exponentielle.

3. Le procédé de la revendication 1 ou de la revendication 2 dans lequel lesdites cellules non adhérentes de mammifères sont cultivées à une pression partielle de l'oxygène dissous inférieure à 0,2 bar (atmosphère) après le stade moyen de la phase de culture exponentielle.

4. Le procédé de la revendication 1, 2 ou 3 dans lequel lesdites cellules non adhérentes de mammifères sont cultivées à une pression partielle de l'oxygène dissous supérieure à 0,02 bar (atmosphère) après le stade moyen de la phase de croissance exponentielle.